# EUROPEAN PATENT APPLICATION

(11) **EP 4 843 504 A1**
(43) Date of publication of application: **25.11.2026**
(21) Application number: 25177718.1
(22) Date of filing: 20.05.2025
(51) Int. Cl.: A61B 6/00, A61B 6/03

(54) **CAMERA FOR MEDICAL IMAGING SYSTEMS**

(71) Applicant: Koninklijke Philips N.V., 5656 AE Eindhoven (NL)
(72) Inventor: WEIß, Steffen, 5656 AG Eindhoven (NL)
(74) Representative: Philips Intellectual Property & Standards

(57) **Abstract**

A medical imaging system for acquiring image data of a subject is provided. The medical imaging system comprises a gantry configured to receive a subject for medical imaging and a subject support configured to move the subject. The gantry has a user interface comprising at least one camera. Further, the medical imaging system is configured to control one or more parameters of the at least one camera such that the subject is in a field-of-view of the at least one camera.

## Description

### FIELD OF THE INVENTION

The present invention generally relates to the field of subject monitoring during medical imaging, and in particular to camera-based subject monitoring for use in medical imaging systems.

### BACKGROUND OF THE INVENTION

In medical imaging systems such as computed tomography (CT), positron-emission tomography (PET)-CT and magnetic resonance imaging (MRI) a camera can be used to monitor subjects undergoing medical imaging. The camera may be used for monitoring the subject motion, pose, position, physiology signals such as breathing and heartbeat or even subject behavior from facial movements. The subject may be monitored during the preparation stage e.g. when the subject is outside the bore of the imaging system as well as during the image acquisition stage e.g. when the subject is inside the bore of the imaging system.

The images captured by the camera are continuously processed by algorithms to determine subject information. However, the outcome of such processing is prone to inaccuracies due to images not having sufficient quality or the camera not being able to capture desired subject information such as subtle subject movements. Furthermore, usage of such cameras with medical imaging systems has constraints as to the placement of the camera in the examination room and interference with the working of the medical imaging system. This restricts the reliability and useability of cameras as subject monitoring instruments in medical imaging systems. Furthermore, adding a camera to an existing medical imaging system requires the addition of the camera, support and camera cables onto or into the cover and chassis of the imaging system which means a major redesign and associated cost. The redesign also has to meet technical requirements, e.g. a camera placement and cable routing not conflicting with design limitations of existing components.

Thus, there is a need for an improved camera system and medical imaging systems that can employ such a camera system for monitoring the subject.

### SUMMARY OF THE INVENTION

The current invention seeks to provide a medical imaging system and associated camera system for monitoring a subject during medical imaging. The current invention, additionally or alternatively, seeks to provide a camera system employed with a medical imaging system for monitoring the subject with increased accuracy and sensitivity regardless of the presence of ambient constraints such as presence of an operator and/or accessory and their movements in the examination room.

Thereto, a medical imaging system for acquiring medical image data of the subject are provided, as well as a user interface for the medical imaging system.

A medical imaging system comprising a gantry configured to receive a subject for medical imaging is provided. The gantry comprises a user interface comprising at least one camera. The medical imaging system further comprises a subject support configured to move the subject wherein the medical imaging system is configured to control one or more parameters of the at least one camera such that the subject is in a field-of-view of the at least one camera.

In an embodiment the one or more parameters is at least one of a focus, a field-of-view and a view direction of the at least one camera.

According to one aspect, the user interface comprising at least one camera is located at a maximal distance from a z-axis of the medical imaging system.

Preferably, the user interface is a touch screen-based user interface.

In a further embodiment, the touch screen comprises one or more recesses to accommodate the at least one camera.

According to another aspect, the medical imaging system is configured to control the one or more parameters of the at least one camera by means of a controller.

In an embodiment, the medical imaging system is configured to control the one or more parameters of the at least one camera by identifying at least one of a subject table position, a subject position, and a subject pose.

In another option, a view axis of the at least one camera is tilted towards the z-axis of the medical imaging system.

In yet another embodiment of the invention the user interface and the at least one camera has at least one common cable for routing data. The data may at least partly comprise video data acquired by the camera. A particular advantage of this embodiment is that separate cables for the camera are not required and additional hardware and costs for routing such cables are avoided. In an even more advantageous example, the cables of the user interface or touch screen may be used for the camera. The video data to and from the camera can be routed using the same cables meant for the user interface.

In a further embodiment, the user interface comprising at least one camera is configured to be separately attached to the medical imaging system.

According to another aspect, a user interface for a medical imaging system is provided. The user interface comprises a touch screen having a touch screen area, the touch screen area is configured for user interaction. The user interface comprises at least one camera wherein the user interface is configured to be attached to a gantry of the medical imaging system such that one or more parameters of the at least one camera is controlled by the medical imaging system.

In an embodiment the medical imaging system is one of a computed tomography, a magnetic resonance or a positron emission tomography system.

The medical imaging system according to one or more of the embodiments above has the advantage that the parameters of the camera can be continuously and seamlessly adapted by the medical imaging system to follow the subject, even on the moving subject table. The camera may be situated farther away from the z-axis of the medical imaging system though closer to the subject than a ceiling mounted camera. Thus, the field-of-view of the camera is unobstructive but still closer to the subject such that it is able to continuously follow the subject on the table and adapt its parameters instantaneously in real-time without any time delay. This results in camera-based monitoring with high sensitivity and accuracy. Additionally, when the camera and the user interface are embodied as a single module, the camera is less likely to be obstructed by frequent operator or accessory movements around the imaging system and an uninterrupted and continuous control of camera parameters is realized when following the subject. Hence, the camera can be used to optimally observe any body part of the subject, such as the subject face, even when the subject is in motion on the subject table or in the presence of operator/accessories in the examination room.

In the medical imaging system according to one or more embodiments above, both the user interface and the camera are farther away from the rotating gantry of the imaging system avoiding any interference with the imaging system and therefore no safety testing is required for the installation of the user interface and the camera.

### BRIEF DESCRIPTION OF THE DRAWINGS

In the following drawings:
Fig. 1 illustrates an imaging arrangement for acquiring medical image data of a subject comprising a medical imaging system according to embodiments of the invention.
Fig. 2 illustrates part of a CT imaging system with a gantry comprising a user interface and a camera.
Fig. 3 illustrates a front view of a medical imaging system comprising a user interface on the gantry according to the invention.
Fig. 4 is a schematic diagram illustrating an assembly of user interface and a camera according to an embodiment of the invention.

### DETAILED DESCRIPTION OF THE EMBODIMENTS

In the examples below, medical image data is acquired for a subject. Such a subject can be a human being, particularly a human patient requiring imaging for medical purposes. However, alternative subjects are also envisaged, for example animals such as research animals, pets, or livestock. In the examples, the medical imaging system is illustrated as a computed tomography (CT) scanner, but other devices such as magnetic resonance (MRI), positron emission tomography (PET), PET-CT may also be used to acquire the medical image data.

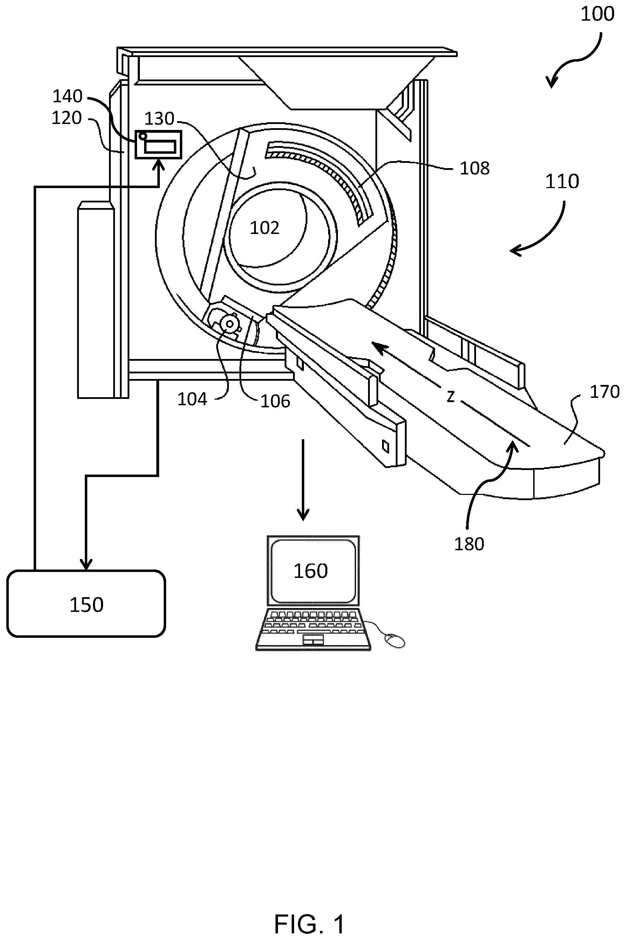

Fig. 1 illustrates an imaging arrangement 100 for acquiring medical image data of a subject.

The imaging arrangement 100 comprises a medical imaging system 110. The medical imaging system in this exemplary embodiment is a computed tomography scanner. The computed tomography imaging scanner 110 includes a stationary gantry 120 and a rotating gantry 130, which is rotatably supported by the stationary gantry 120. When the system is in operation, the rotating gantry 130 rotates around an examination region 102 about a longitudinal or z-axis. A radiation source 104, such as an x-ray tube, is supported by and rotates with the rotating gantry 130 and emits radiation that traverses the examination region 102. A source collimator 106 collimates the emitted radiation to form a generally fan, wedge, or cone shaped radiation that traverses examination region 102.

A subject support or table 170, such as a bed or couch, is provided to support an object or subject to be imaged in examination region 102. The subject support 170 is movable along a z-axis 180 in coordination with the rotation of the rotating gantry 130 to facilitate the desired scanning trajectory. The z-axis 180 corresponds to the direction along the length of the subject support in the direction of movement, which is a direction that is parallel to the axis of rotation of the rotating gantry 130.

A radiation sensitive detector array 108 detects radiation emitted by the radiation source 104 that traverses the examination region 102 and generates projection image data indicative of the detected radiation. The imaging arrangement 100, optionally further comprises an image reconstruction system, which reconstructs the projection data and generates volumetric image data indicative of the examination region 102, including structure of an object or subject disposed therein. One or more images can be generated from the volumetric image data. The imaging arrangement 100 also comprises an optional display 160 for displaying the one or more generated images.

The medical imaging system 110, in this example CT imaging system, communicates with a controller 150. The controller may be implemented by means of one or more processors and suitable hardware modules. In an embodiment of the invention the controller may comprise one or more memory and a computer program code. The controller 150 may be either a component within the imaging system 110 or may be external to the imaging system 110. When external to the imaging system, the imaging system communicates with the controller 150 through either a wired or a wireless communication means. The controller 150 may be configured to control the operation of the imaging system 110 to acquire image data of the subject. The operation may include controlling imaging parameters, monitoring the imaging process, for example the subject position and the imaging parameters, and displaying the generated images. In other embodiments the controller may be configured to control the movement of the subject support 170 into and out of the examination region 102 of the gantry 120.

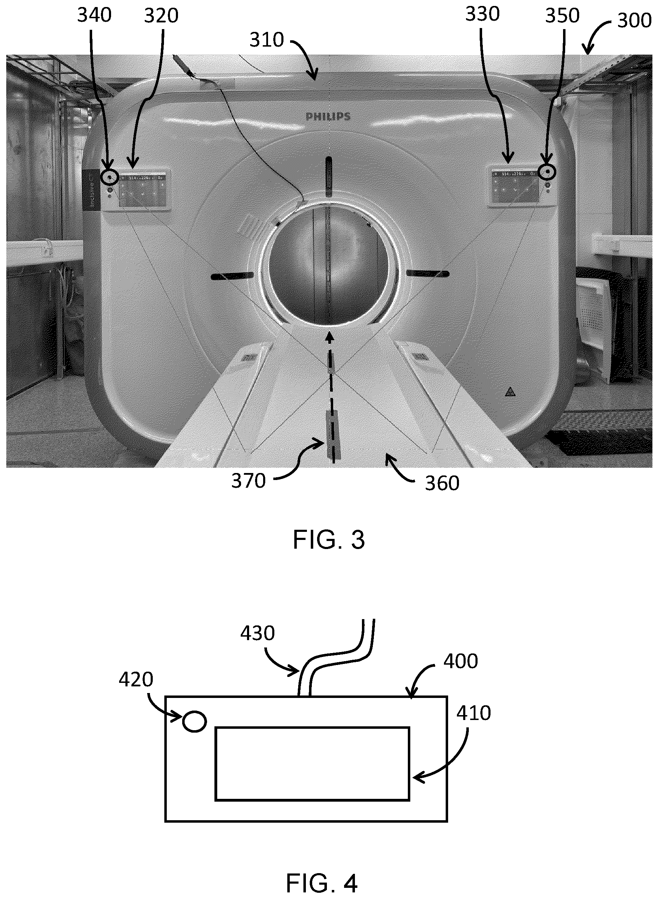

Referring to the schematics of Fig. 1, the CT imaging system 110 further comprises a user interface 140 mounted on the gantry 120 of the imaging system 110. In an embodiment, the user interface may be a touchscreen-based user interface. The user interface comprises one or more cameras (not shown). The one or more cameras may be mounted on the user interface such that the user interface and camera are a single module. The detailed construction of the user interface and camera will be further elaborated with reference to Fig. 4. In an exemplary embodiment of the invention, the medical imaging system 110 is configured to control one or more parameters of the camera by means of the controller 150. The parameters of the camera may be controlled such the subject is in the field-of-view of the at least one camera. The detailed working of the controller in conjunction with the medical imaging system and the user interface comprising the camera will be further elaborated with reference to Fig. 3.

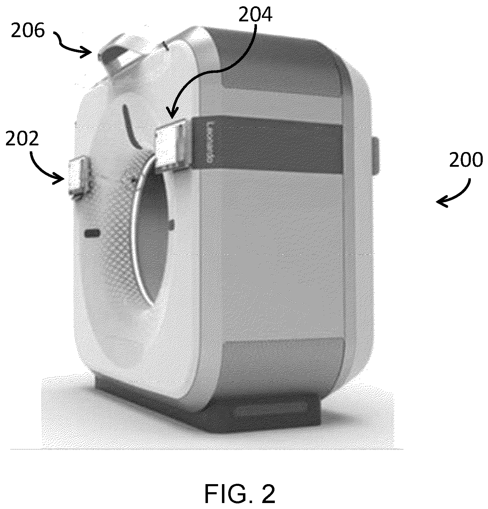

Fig. 2 shows part of an imaging device, in this example a CT imaging system, comprising a gantry wherein a user interface and a camera are shown mounted on the gantry. The user interface, in this example touch screens 202 and 204, are mounted on the gantry 200. Also, a camera 206 is shown mounted on top of the gantry. Configurations where a camera is mounted with a top view of the subject, such as ceiling-mounted camera, are known. As can be seen from the figure, the touch screens 202 and 204 and camera 206 are separate modules, and the camera is projected outward to obtain a field-of-view covering the subject table (not shown) till the foot end of the subject prior to entering the gantry 200.

Fig. 3 shows a medical imaging system in the form of a CT system 300 according to an exemplary embodiment of the invention. A CT gantry 310 is shown for receiving a subject for CT imaging. A subject table or support 360 supports the subject for medical imaging. The subject support 360 is moved into and out of the bore and the imaging system is configured to continuously monitor the subject on the subject support both outside and inside the bore. The gantry 310 comprises user interfaces 320 and 330, the user interfaces are mounted on the gantry. Although Fig. 3 shows two user interfaces mounted on the left and right side of the bore, and on the front side of the gantry having two interfaces is not essential and any number of user interfaces may be mounted on the gantry without deviating from the scope of the invention. One or more user interfaces may also be mounted on the back side of the gantry instead of or in addition to one or more user interface on the front side. In an embodiment only one user interface is mounted on the gantry. In further embodiments the user interface comprising the camera is located at a maximal distance from the z-axis 370 of the imaging system. According to a preferred example, the user interfaces 320 and 330 are touch screen-based user interfaces. Alternatively, or additionally, the user interfaces 320 and 330 can be button operated. The touch screen-based user interface is configured to receive a user or operator input through touch-based interactions. The user or operator input may be used to control the working of the CT imaging system. The cameras 340 and 350 are mounted on the user interfaces 320 and 330 respectively as shown in Fig 3. In an embodiment of the invention the parameters of the cameras 340 and 350 are controlled through the user interface. Thus, the user interface or touch screen may be used as a basis for mounting the camera and also for controlling the camera parameters.

In some examples, the user interface comprising the camera is a single module and is configured to be separately mounted or attached to the medical imaging system, for example to the gantry of the imaging system. In further examples, the module with the user interface and camera may comprise appropriate firmware, hardware and memory and the parameters and functioning of the user interface and camera are controllable externally. The module attached to the imaging system may be configured to communicate with the controller of the medical imaging system through either a wired or a wireless means of communication. Thus, in the above examples the module with the user interface and camera may be an independent entity and is configured to be controlled externally.

As shown in Fig. 3 the user interfaces 320, 330 comprising one or more cameras 340 and 350 are farther away from the z-axis 370 of the imaging system. In an embodiment of the invention the user interface comprising the camera is mounted on the gantry at a maximal distance from the z-axis of the medical imaging system. For example, in Fig. 3 the left-side user interface 320 has the camera 340 mounted on the left corner of the user interface 320 such that the camera 340 is located at the outer periphery of the gantry 310. Similarly, the right-side user interface 330 has the camera 350 mounted on the right corner of the user interface 330 such that the camera is located at the outer periphery of the gantry 310. In a further embodiment the view axis of the camera is tilted toward the z-axis of the imaging system. In this way the camera is able to view the end of the subject table. Since the camera is farther away from the z-axis of the imaging system, interference with the imaging system is minimized or even completely avoided. Furthermore, by mounting the user interface and camera as a single module at a maximal distance from the z-axis of the imaging system besides avoiding additional effort and costs of mounting the cameras on the gantry there is less interference in the field-of-view of the camera by frequent operator and/or accessory movements. This enables a more reliable, accurate and consistent control of camera parameters by imaging system. This is particularly important when a specific body part of the subject, such as face, is to be followed by the camera to derive subtle subject information such as subject behavior or emotion.

In yet another embodiment of the invention the CT imaging system controls the user interface-based cameras and adapts one or more parameters of the camera such as its focus e.g. focal depth, field-of-view e.g. focal length and/or its view direction to optimally follow the subject on the table while the table is moving up and down or into the bore of the imaging system. The CT system may control the above camera parameters by means of the controller of the CT system. The construction and working of the controller are already described above with reference to Fig. 1. The controller communicates with the CT system and also with the camera and the user interface. When the camera parameters are controlled the controller of the imaging system exploits the knowledge of current table position and/or subject pose or position. The current table position and/or subject pose or position are provided as inputs to the controller. The controller receives the inputs and determines optimal parameters of the camera at a particular point in time. The control and adaptation of camera parameters may even be performed real-time by the controller of the CT system. In an embodiment of the invention the defining of camera parameters by receiving the above input may also be performed by a software and/or artificial intelligence/machine learning algorithm. Hence, the camera mounted on the user interface operates with the imaging system to optimally follow and observe any body part of the subject.

Usually, the examination room has a number of objects such as pillows, straps, subject supports etc. placed in the vicinity of the imaging system to prepare the subject for imaging. These objects are frequently moved by the operator from one position to the other during subject preparation. Above that, there is operator movement in the examination room. Such movement of objects and operators in the examination room frequently hinders the camera view of the subject. With the arrangement of the camera and user interface according to the invention, obstruction in the field-of-view of the camera is minimized and adaptation of camera parameters is consistent and less prone to inaccuracies. Hence, it is possible to optimally observe the face or any body part of the patient with high sensitivity and continuity even in the presence of ambient movements of object and operator.

Fig. 4 is an exemplary embodiment showing an assembly of the camera and the user interface. In the figure a user interface 400 with screen area 410 is shown. In an embodiment of the invention the user interface 400 is a touch screen-based user interface. The touch screen-based user interface is configured for receiving user input by way of touch-based input on the screen area 410. In some examples, a camera 420 is mounted on the user interface at one corner of the user interface 400 as shown in Fig. 4. In this example, by mounting the camera 420 at the left corner of the user interface 400 which is configured to be mounted on the left side of the gantry puts the camera at the periphery of the gantry and further away from the z-axis of the imaging system. Similarly, a user interface which is configured to be mounted on the right side of the gantry, the camera may be mounted on the right side of the user interface such that the camera is located at the periphery of the gantry. Thus, by appropriately positioning the camera on the user interface the camera may be located at a maximum distance from the z-axis of the imaging system but still mounted on the gantry of the imaging system. In another example, the camera 420 may be mounted at any other different location on the user interface 400. In further examples, one or more additional camera may be mounted at other locations on the user interface. The touch screen may comprise one or more recesses to accommodate one or more cameras. The camera is mounted in the recess and configured such that the controller is able to adapt the parameters of the camera. The one or more cameras and the user interface are combined as a single module, and they may have common cables for routing interface data and/or video data. In the embodiment of Fig. 4, a common cable 430 is shown which is used for routing the data to and from the camera and the user interface. In a further embodiment of the invention existing cables of the user interface may be used to additionally transmit the video data acquired by the camera.

In a further embodiment, the user interface comprising one or more cameras may be configured to be separately attached to the medical imaging system, for example to the gantry of the imaging system. Therefore, by mounting the user interface and camera as a single module with common cables not only provides ease of mounting the module to the imaging system but also simplifies the control of the module by the controller of the imaging system. The imaging system controller, instead of controlling the camera and user interface separately at two different locations, is now able to control the camera and the user interface as one entity located at a common location. It is a further advantage of mounting the user interface and camera as a single module, that medical imaging systems already comprising a user interface but not, yet a camera can be upgraded by a camera with much lower effort compared to adding a camera separately. The latter would require the separate dedicated design of a camera support and cable routing which may be complicated by space requirements of other components of the medical imaging system. The replacement of the existing user interface with a new user interface comprising a camera avoids this effort.

In some embodiments of the invention, a user interface for the medical imaging system is provided. The user interface comprises a touch screen having a touch screen area, the touch screen area configured for user interaction. The user interface further comprises at least one camera. The user interface is configured to be attached to a gantry of the medical imaging system such that one or more parameters of the at least one camera is controlled by the medical imaging system. In further examples, the user interface and camera may comprise appropriate firmware, hardware and memory such that the parameters and functioning of the user interface and camera can be controlled by the imaging system. Thus, by mounting user interface and camera on a common platform as a single module synergy between the user interface and camera can be maximized.

Any of the method steps disclosed herein may be recorded in the form of a computer program comprising instructions which when executed on a processor cause the processor to carry out such method steps. The instructions may be stored on a computer program product. The computer program product may be provided with dedicated hardware as well as hardware capable of executing software in association with appropriate software. When provided by a processor, the functions can be provided by a single dedicated processor, by a single shared processor, or by a plurality of individual processors, some of which can be shared. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid-state memory, magnetic tape, a removable computer diskette, a random-access memory "RAM", a read-only memory "ROM", a rigid magnetic disk and an optical disk. Current examples of optical disks include compact disk - read only memory "CD-ROM", compact disk - read/write "CD-R/W", Blu-Ray^{™} and DVD. Examples of a propagation medium are the Internet or other wired or wireless telecommunication systems.

Variations to the disclosed embodiments can be understood and effected by those skilled in the art in practicing the claimed invention, from a study of the drawings, the disclosure, and the appended claims. It is noted that the various embodiments may be combined to achieve further advantageous effects.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A medical imaging system comprising:
- a gantry configured to receive a subject for medical imaging, the gantry further comprising:
a user interface comprising at least one camera; and
- a subject support configured to move the subject;
wherein the medical imaging system is configured to control one or more parameters of the at least one camera such that the subject is in a field-of-view of the at least one camera.

2. The medical imaging system according to claim 1, wherein the one or more parameters is at least one of a focus, a field-of-view and a view direction of the at least one camera.

3. The medical imaging system according to claims 1 or 2, wherein the user interface comprising at least one camera is located at a maximal distance from a z-axis of the medical imaging system.

4. The medical imaging system according to any of claims 1-3, wherein the user interface is a touch screen-based user interface.

5. The medical imaging system according to claim 4, wherein the touch screen comprises one or more recesses to accommodate the at least one camera.

6. The medical imaging system according to any of claims 1-5, wherein the medical imaging system is configured to control the one or more parameters of the at least one camera by means of a controller.

7. The medical imaging system according to any of claims 1-6, wherein the medical imaging system is configured to control the one or more parameters of the at least one camera by identifying at least one of a subject table position, a subject position, and a subject pose.

8. The medical imaging system according to any of claims 1-7, wherein a view axis of the at least one camera is tilted towards the z-axis of the medical imaging system.

9. The medical imaging system according to any of claims 1-8, wherein the user interface and the at least one camera has at least one common cable for routing data.

10. The medical imaging system according to any of claims 1-9, wherein the user interface comprising at least one camera is configured to be separately attached to the medical imaging system.

11. A user interface for a medical imaging system comprising:
- a touch screen having a touch screen area, the touch screen area configured for user interaction; and
- at least one camera;
wherein the user interface is configured to be attached to a gantry of the medical imaging system such that one or more parameters of the at least one camera is controllable by the medical imaging system.

12. The user interface according to claim 11, wherein the one or parameters of the at least one camera is controllable by means of a controller.

13. The user interface according to claims 11 or 12, wherein the one or more parameters is at least one of a focus, a field-of-view and a view direction of the at least one camera.

14. The medical imaging system according to any of claims 1-13, wherein the medical imaging system is one of a computed tomography, a magnetic resonance or a positron emission tomography system.
